Europäisches Patentamt

European Patent Office

Office européen des brevets

(19)

(11) Veröffentlichungsnummer: **0 390 067**

**A1**

## (12) EUROPÄISCHE PATENTANMELDUNG

(21) Anmeldenummer: 90105799.2

(22) Anmeldetag: 27.03.90

(51) Int. Cl.⁵: **C12N 15/12, C12P 21/02, A61K 37/64**

(30) Priorität: 30.03.89 DE 3910240

(43) Veröffentlichungstag der Anmeldung:
03.10.90 Patentblatt 90/40

(84) Benannte Vertragsstaaten:
**AT BE CH DE DK ES FR GB GR IT LI LU NL SE**

(71) Anmelder: **BEHRINGWERKE Aktiengesellschaft**
**Postfach 1140**
**D-3550 Marburg 1(DE)**

(72) Erfinder: **Römisch, Jürgen, Dr.**
**Am Kähnelplatz 6**
**D-3550 Marburg(DE)**
Erfinder: **Grote, Mathias, Dr.**
**Gladenbacher Weg 65**
**D-3550 Marburg(DE)**
Erfinder: **Amann, Egon, Dr.**
**Sachsenring 8**
**D-3550 Marburg(DE)**
Erfinder: **Stüber, Werner, Dr.**
**Cölber Weg 12**
**D-3551 Lahntal(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) **PP4-delta, seine Herstellung und Verwendung.**

(57) Die Erfindung betrifft PP4-delta, seine Herstellung und Verwendung. PP4-delta ist ein gegenüber dem antikoagulatorischen Protein PP4 am C-terminalen Ende um 13 Aminosäuren verkürztes Protein und besitzt überraschenderweise eine im Vergleich zum Ausgangsprotein höhere Aktivität im Thromboplastin-Inhibitionstest.

EP 0 390 067 A1

## PP4-delta, seine Herstellung und Verwendung

Die Erfindung betrifft PP4-delta, seine Herstellung und Verwendung. PP4-delta ist ein gegenüber PP4 am C-terminalen Ende um 13 Aminosäuren verkürztes Protein und besitzt überraschenderweise eine im Vergleich zum Ausgangsprotein höhere Aktivität im Thromboplastin-Inhibitionstest.

Sowohl die Physiologie der Hämostase als auch die damit eng verknüpfte Immunabwehr, einschließlich der Aktivierung von Blutzellen, wurden in den letzten Jahren intensiv untersucht. Danach wird die plasmatische Gerinnung durch Kontakt von Blut mit Fremdoberflächen oder durch die Freisetzung von Thromboplastin nach Zelläsionen ausgelöst; sie verläuft im Prinzip gemäß Enzym-Substrat-Reaktionen. Dabei werden die einzelnen Gerinnungsfaktoren in einer Kaskadenreaktion und über eine limitierte Proteolyse aus der Proenzymform in die aktive Form überführt. Diese Umwandlung erfolgt unter Beteiligung zellulärer Blutbestandteile, vorwiegend der Thrombozyten, die mit ihren Phospholipid-Bilayers die Reaktionsoberfläche für die plasmatische Gerinnung abgeben. Der Prozeß der Hämostase wird durch Kalzium und Akzelerator-Proteine wesentlich beschleunigt.

Als Regulatoren des Hämostaseablaufes fungieren sowohl Protease-Inhibitoren, die mit den aktivierten Gerinnungsenzymen stabile, inaktive Komplexe bilden, wie z.B. das Thrombin oder der Faktor Xa mit ATIII als auch proteolytische Enzyme, wie das Protein C, das die Faktoren Va und VIIIa proteolytisch inaktiviert.

Durch die Inaktivierung dieser beiden Faktoren, die zu den Akzeleratoren gehören, wird der Gerinnungsablauf stark verlangsamt.

Ein alternatives Regulationsprinzip stellen Proteine dar, die die für den Gerinnungsablauf essentiellen Phospholipide zu binden vermögen (Reutelingsperger, C.P.M et al. (1985) Eur.J.Biochem. 251, 625-629).

Mit dem Protein PP4 wurde ein solcher Eiweißkörper erstmals von Bohn et al. (Arch. Gynaekol. 236, (1985), 225-233) isoliert, das mit dem kürzlich beschriebenen "VAC"-Protein (Maurer-Fogy et al. (1988) Eur.J.Biochem. 174, 583-592) identisch ist.

Die antikoagulatorische Wirkung von PP4 bzw. PP4-delta ist darauf zurückzuführen, daß diese Proteine sowohl die Plättchenaggregation (primäre Hämostase) inhibieren als auch die Schritte beim Ablauf der plasmatischen Gerinnung, an der Phospholipide und Kalzium teilhaben. Die anti-entzündliche Wirkung ist im Prinzip auf die Hemmung der Phospholipase A2 zurückzuführen.

Zur Klonierung und Sequenzierung von PP4-cDNA (Grundmann et al. (1988) Proc.Natl.Acad.Sci. 88, 3708-3712) wurden von partiellen Aminosäuresequenzen des aus Plazenta isolierten Proteins Oligonukleotid-Sonden abgeleitet und eine cDNA-Bank, hergestellt aus mRNA von Humaner Plazenta, einem Screening unterzogen. Dabei wurden neben PP4-Klonen ebenfalls solche entdeckt, deren cDNAs für ein dem PP4 strukturell verwandtes Protein kodieren, PP4-X genannt (Grundmann et al. a.a.O.), das dem PP4 vergleichbare physiologische Wirksamkeit besitzt.

Sowohl PP4 als auch PP4-X gehören einer Gruppe von Proteinen an, die als Lipocortine (Glucocorticoid-induzierte Phospholipase-hemmende Proteine; Cirino, G. et al. (1987) Br.J.Pharmacol. 92, 521) oder Calpactine (Calziumabhängige Phospholipid- und Aktin-bindende Proteine; Kristensen, T. et al. (1986) Biochemistry 25, 4497-4503) bezeichnet werden. Diese Proteine, z.B. Lipocortin I und II als auch PP4, PP4-X und PP4-delta haben neben ihrer antikoagulatorischen auch Phospholipase A2-hemmende Wirkung.

Die Mitglieder dieser Familie zeigen bezüglich ihrer Primärstrukturen hohe Homologie, die besonders in vier zentralen repetitiven Sequenzen ausgeprägt ist. Diese "Repeats" fungieren vermutlich als Kalzium-Bindungsstellen und sind offenbar an der Bindung von Phospholipiden beteiligt. Die terminalen Sequenzen dagegen sind höher variabel, wobei die aminoterminalen Bereiche offenbar zur Regulation der physiologischen Wirksamkeit dienen. Wie für Calpactin I gezeigt wurde, wird die Affinität zu Phospholipiden durch Phosphorylierung von Tyrosin- und/oder Serinresten in der Nähe des N-Terminus stark verringert. Dem carboxyterminalen Bereich konnten dagegen bisher keine funktionellen Eigenschaften zugeschrieben werden.

Durch Deletion der für die C-terminalen 13 Aminosäuren kodierenden Region von PP4 und Expression in E.coli wurde ein verkürztes PP4 (bezeichnet als PP4-delta) hergestellt, das überraschenderweise eine erhöhte antikoagulatorische Aktivität gegenüber dem nicht-deletierten PP4 aufweist.

Als anti-entzündlich wirksame Proteine hemmen die Lipocortine das an der Innenseite der Plasmamembran lokalisierte Enzym Phospholipase A2, das beispielsweise bei Septikämien aktiviert wird und aus Lipiden der Membran Arachidonsäuremoleküle freisetzt, die wiederum Ausgangsprodukte für die Synthese von Entzündungsmediatoren vom Typ der Prostaglandine und Leukotriene sind.

Die meisten der derzeit angewendeten anti-inflammatorisch wirksamen Pharmazeutika, wie Aspirin oder Indomethazin, inhibieren die Cyclooxygenase, ein Enzym der Protaglandin-Synthese, und damit eine der möglichen Umsatzreaktionen der Arachidonsäure zu Entzündungsmediatoren. Im Gegensatz dazu inhibieren Glucocorticoide die Freisetzung von Arachidonsäure aus Membranlipiden, wahrscheinlich durch Aktivierung bzw. Stimulation der Synthese von Proteinen der Lipocortin-Familie. Es ist bekannt, daß bei der Behandlung inflammatorischer Krankheiten jedoch eine Reihe von Nebenreaktionen auftreten können, wie z.B. Hyperglykämien oder Hyperlipidämien. Die direkte Applikation eines PP4-, PP4-X- oder vorzugsweise PP4-delta-Präparates würde diese unerwünschten Effekte vermeiden.

Da bei Septikämien neben den Makrophagen und Monozyten auch das lipolytische System aktiviert wird und damit verbunden Entzündungsmediatoren synthetisiert werden, können schwere hämostatische Störungen auftreten. Diese gehen mit einer Exposition und intravasaler Ausschüttung von Thromboplastin einher, wodurch eine disseminierte intravasculäre Gerinnung (DIC) ausgelöst werden kann (Prentice, C.R. et al. (1985) Clin. Haematol. 14, 413-442). Zur Begegnung dieses hyperkoagulatorischen Zustandes durch Neutralisation des Thromboplastins wurden im wesentlichen zwei Verfahren beschrieben.

Erstens ein therapeutisches Verfahren mittels Applikation eines Enzyms, der "Thromboplastinase" (Gollub, S. et al. (1962) Thromb. Diath. Haemorh. 7, 470-479), die aufgrund ihrer lipolytischen Aktivität den Gewebsfaktor inaktiviert, daneben jedoch auch Zellmembran-schädigende Wirkung entfalten kann.

Zweitens ein therapeutisches Verfahren, nämlich Einsatz von gegen das Thromboplastin-Apoprotein gerichteter monoklonaler Antikörper (Carson, S.D. et al. (1985) Blood 66, 152-156), das jedoch den Nachteil aufweist, daß solche Antikörper beim derzeitigen Stand der Technik nur aus nicht-humanen Organismen gewonnen werden können.

Dagegen kann bei Applikation von PP4, PP4-X oder vorzugsweise von PP4-delta in einem homologen System therapiert werden, ohne daß es zu einem Verbrauch oder proteolytischer Inaktivierung von Gerinnungsfaktoren und damit Blutungen kommt.

Die Erfindung betrifft folglich PP4-delta, seine gentechnische Herstellung sowie seine Verwendung als Arzneimittel, vorzugsweise als Hämostyptikum. Die Erfindung ist ferner in den Beispielen und den Patentansprüchen beschrieben.

**Beispiel: Konstruktion eines um die 13 C-terminalen Aminosäuren verkürzten PP4 Proteins ("PP4-delta")**

Das von Grundmann et al. (a.a.O.) beschriebene 5235 Basenpaar große Plasmid pMc5-8-PP4-NcoI diente als Ausgangsplasmid für eine Mutagenese nach der bekannten "gapped duplex" Methode (Kramer et al. (1984) Nucl. Acids. Res. 12, 9441-9256). Zur Mutagenese wurde ausgehend von einer pMc5-8-PP4-NcoI Plasmid-haltigen Bakterienkolonie zunächst ein DNA-Einzelstrang in Form eines rekombinanten F1 Bakteriophagen isoliert. Dieses ringförmige Einzelstrangmolekül wurde sodann mit einem 3765 Basenpaar großen EcoRI-XbaI Fragment, welches nach entsprechendem Verdau des Mutagenesevektors pMa-5-8 erzeugt und isoliert worden war, zusammengebracht und den bekannten Schritten der Mutagenese nach der "gapped duplex" Methode unterzogen, wobei folgendes Oligodeoxynukleotid verwendet wurde:

5´ AAGAGCTTTCTTTTAGTCCCCAGATGT 3´

Nach erfolgter Mutagenese wurden Ampicillin-resistente Kolonien mit F1 Bakteriophagen infiziert, Einzelstrang-DNA isoliert und der mutagenisierte DNA-Abschnitt nach der Dideoxysequenzierungsmethode (Sanger et al. (1977) Proc.Natl.Acad.Sci. USA 74, 5463-5467) sequenziert, wobei folgender Sequenzierungsprimer verwendet wurde:

5´ AGGGAGCTCTTCCGTGACA 3´

Ein Bakterienklon, dessen F1 Einzelstrang-Analyse die gewünschte Mutation aufwies, wurde vermehrt und Plasmid DNA nach bekannten Methoden isoliert. Dieses als pMA-5-8-PP4-NcoI-delta bezeichnete Plasmid wurde mit NcoI und HindIII verdaut, das 1307 Basenpaar große Fragment isoliert und in den mit den gleichen Enzymen behandelten Expressionsvektor pTrc99A (Amann et al. (1988), Gene 69, 301-315) ligiert. Das resultierende Plasmid pTrc99A-PP4-delta ist in der Lage, ein um 13 Aminosäuren verkürztes PP4 Protein herzustellen, wobei sich die DNA-Sequenzen kodierend für die C-Termini bzw. die C-terminalen Enden von PP4 und PP4-delta im Vergleich folgendermaßen darstellen:

PP4:

Ser Gly Asp Tyr Lys Lys Ala Leu Leu Leu Leu Cys
Gly Glu Asp Asp Stop
TCT GGG GAC TAT AAG AAA GCT CTT CTG
CTG CTC TGT GGA GAA GAT GAC TAA

PP4-delta:

Ser Gly Asp Stop
TCT GGG GAC TAA AAG AAA GCT CTT CTG
CTG CTC TGT GGA GAA GAT GAC TAA

Das neue Plasmid pTrc99A-PP4-delta umfaßt ebenso wie das Plasmid pTrc99A-PP4 (Grundmann et al., a.a.O) 5427 Basenpaare, exprimiert nach Induktion des trc-Promoters aber aufgrund des eingeführten Stopcodons TAA ein um 13 Aminosäuren verkürztes PP4 Protein. Dieses neue, als PP4-delta bezeichnete Protein reagiert im Westernblot und in Immunpräzipitationsversuchen mit Antiseren gegen PP4 und kann analog zum Reinigungsschema von PP4 dargestellt werden. Nach gelelektrophoretischer Auftrennung und Coomassieblaufärbung von PP4 und PP4-delta kann der Unterschied im Molekulargewicht beider Proteine (PP4: ca. 32 kD, PP4-delta: ca. 30 kD) beobachtet werden. Es wurde nun gefunden, daß nach Reinigung der beiden Proteine aus Escherichia coli Zellextrakten PP4-delta im Vergleich zu PP4 eine höhere spezifische Aktivität im Thromboplastin-Inhibitionstest aufweist.

**Ansprüche**

1. PP4-delta Protein mit erhöhter Aktivität im Thromboplastininhibitionstest gegenüber PP4, dadurch gekennzeichnet, daß C-terminale Aminosäuren deletiert sind.

2. PP4-delta Protein nach Anspruch 1, dadurch gekennzeichnet, daß die 13 C-terminalen Aminosäuren deletiert sind.

3. PP4-delta Protein nach Anspruch 1 oder Anspruch 2 als Arzneimittel.

4. Therapeutische Zubereitung, enthaltend PP4-delta Protein nach Anspruch 1 oder 2 und inertes Trägermaterial.

5. Verfahren zur Herstellung von PP4-delta Protein nach Anspruch 1 oder 2, dadurch gekennzeichnet, daß eine für PP4-delta Protein kodierende cDNA in ein Expressionssystem eingebracht und dort zur Expression gebracht wird.

6. Verwendung von PP4-delta Protein nach Anspruch 1 oder 2 für die Herstellung eines Arzneimittels zur Behandlung von Gerinnungsstörungen.

Patentansprüche für folgende Vertragsstaaten: Es, Gr.

1. Verfahren zur Herstellung von PP4-delta Protein mit erhöhter Aktivität im Thromboplastintest durch Einbringen einer für PP4-delta kodierenden cDNA in ein Expressionssystem und anschließende Expression der genannten cDNA, dadurch gekennzeichnet, daß für C-terminale Aminosäuren kodierende Bereiche deletiert sind.

2. Verfahren nach Anspruch 1, dadurch gekennzeichnet, daß der für die 13 C-terminalen Aminosäuren kodierende Bereich deletiert ist.

3. Verfahren zur Herstellung eines Arzneimittels, dadurch gekennzeichnet, daß PP4-delta Protein, hergestellt nach Anspruch 1 oder 2, mit einem inerten Trägermaterial gemischt wird.

Europäisches
Patentamt

**EUROPÄISCHER RECHERCHENBERICHT**

Nummer der Anmeldung

EP 90 10 5799

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 293 567 (BOEHRINGER INGELHEIM) * Seite 8, Zeile 57 - Seite 9, Zeile 7; Ansprüche * --- | 1-6 | C 12 N 15/12 C 12 P 21/02 A 61 K 37/64 |
| P,A | EP-A-0 339 285 (RORES INTERNATIONAL) * Seite 4, Zeilen 1-9; Ansprüche; Abbildung 1 * ----- | 1-6 | |

RECHERCHIERTE
SACHGEBIETE (Int. Cl.5)

C 12 N

Der vorliegende Recherchenbericht wurde für alle Patentansprüche erstellt

| Recherchenort | Abschlußdatum der Recherche | Prüfer |
|---|---|---|
| DEN HAAG | 27-06-1990 | HUBER A. |